# EUROPEAN PATENT SPECIFICATION

(11) **EP 0 646 376 B1**
(45) Date of publication and mention of the grant of the patent: **10.05.2000**
(21) Application number: 94112427.3
(22) Date of filing: 09.08.1994
(51) Int. Cl.: A61K 38/16, A61K 39/39

(54) **Use of histamine-added gamma-globulin for the manufacture of an immunomodulating and antiinflammatory agent**
Verwendung von Histamin-gamma-globulin zur Herstellung eines immunomodulierenden und antiinflammatorischen Agens
Utilisation de l'histamine-gamma-globuline pour la fabrication d'un agent immunomodulateur et anti-inflammatoire

(30) Priority: 09.08.1993 JP 21804393
(43) Date of publication of application: 05.04.1995
(73) Proprietor: NIPPON ZOKI PHARMACEUTICAL CO., LTD., Chuo-ku, Osaka (JP)
(72) Inventor: Yoshii, Haruo, c/o Inst. of Bio-Active Science, Yashiro-cho, Katoh-gun, Hyogo (JP); Fukata, Yuriko, c/o Inst. of Bio-Active Science, Yashiro-cho, Katoh-gun, Hyogo (JP)
(74) Representative: Hansen, Bernd, Dr. Dipl.-Chem.

(56) References cited:
- CHEMICAL ABSTRACTS, vol. 67, no. 5, 1967, July 31, Columbus, Ohio, USA A. GETLIK et al. "Long-term investigation of the sensitivity threshold limit to acetylcholine during the treatment of pneumoallergoses by histaglobin" pages 1929-30, no. 20 482v; & Cesk.Pediat. 22(3), 211-17 (1967)
- CHEMICAL ABSTRACTS, vol. 115, no. 3, 1991, July 22, Columbus, Ohio, USA Z.P. VOLOKHOVSKAYA et al. "Histaglobulin and changes in conjunctival structure in vernal conjunctivitis" page 43, no. 21 847q; & Zdravookhr. Turkm. 1990, (10), 15-18
- CHEMICAL ABSTRACTS, vol. 112, no. 13, 1990, March 26, Columbus, Ohio, USA T. HIGASHIGUCHI et al. "Basic Studies on nebulizer therapy with histaglobin. Systemic effects and histological findings of nasal and tracheal mucosa in guinea pigs exposed to histamine- added guinea pig gamma- globulin" page 50, no. 111 828b & Yakuri to Chiryo 1989, 17(6), 2549-62

## Description

### Detailed Description of the Invention:

The present invention relates to a novel use of a histamine-added gamma-globulin as a pharmaceutical and, more particularly, it relates to an immunomodulating agent, a suppressive agent to hypereosinophilicity and an antiinflammatory agent containing the histamine-added gamma-globulin as an effective component.

The product used in the present invention is known as a histamine-added gamma-globulin preparation, and exhibits the action of recovering the histamine fixing ability which is lowered in the patients suffering from allergy and asthma and is used as an agent for nonspecific hyposensitizing therapy for bronchial asthma, allergic rhinitis and allergic skin diseases such as urticaria, chronic eczema, atopic dermatitis, etc. It has been also known that this agent exhibits a suppressive action to a histamine liberation and, since it has no side action of antihistaminics and adrenocortical hormones used as symptomatic remedies, it has been widely used as a pharmaceutical agent with high safety.

An object of the present invention is thus the use of histamine-added γ-globulin for the manufacture of a medicament effective as an immunomodulating agent, a hypereosinophilicity suppressive agent and an antiinflammatory agent.

The present inventors have conducted an extensive investigation on said histamine-added gamma-globulin and found its novel pharmacological action, i.e. an immunomodulating action and a suppressive action to hypereosinophilicity, whereby the present invention has been achieved.

Histamine-added gamma-globulin, the effective component of the present invention can be manufactured by an adequate mixing of a gamma-globulin component with a histamine component. Human gamma-globulin used in the present invention may be prepared from serum or from placenta plasma or the like by conventional means while, with respect to a histamine component, free histamine or a pharmacologically-acceptable salt thereof (e.g. hydrochloride, phosphate, picrate, etc.) may be utilized. In the manufacture of the product of the present invention, 100 to 200 mg (preferably 5 to 50 mg) of gamma-globulin may, for example, be mixed with 0.01 to 2 micrograms (preferably 0.05 to 0.5 microgram) of histamine component by a suitable means.

The product of the present invention is mostly used as an injection and, therefore, it can be made into a pharmaceutical composition as an isotonic solution using distilled water for injection or a physiological saline solution. In its manufacture, additives such as auxiliary solubilizers, isotonizing agents, stabilizers, buffers, preservatives, etc. may be used in addition to the gamma-globulnine component and the histamine component. Examples of the applicable additives are citric acid, sodium benzoate, glycine, sodium sulfite, sodium bisulfite, sodium pyrosulfite, sodium thiosulfate, cysteine hydrochloride, phosphates, sodium ascorbate, sodium chloride, sodium bicarbonate, etc.

Further, the product of the present invention may be prepared as an injectable preparation which is dissolved upon actual use. Thus, each of the components may be mixed in a dry state or a mixed solution is filled in vials or the like followed by a freeze-drying. In the manufacture of the dry preparation for injection, fillers such as glucose, mannitol and sorbital may, if necessary, be added in addition to the above-mentioned additives. An example of the dry preparation for injection is a histamine-added immunized human globulin preparation which has been used in clinical fields.

An example of manufacture of the histamine-added gamma-globulin is as follows. Incidentally, mice were used as experimental animals in the following pharmacological tests and, accordingly, mouse gamma-globulin was used in place of human gamma -globulin. Thus, a mouse gamma-globulin and histamine dihydrochloride were dissolved in a distilled water in the following mixing ratio, the solution was stirred at room temperature for two hours, freeze-dried and, upon use, dissolved by adding a physiological saline solution thereto

| Product of the Present Invention | Amount of Mouse gamma-Globulin | Amount of Histamine.2HCl |
|---|---|---|
| HG50 | 5.3 mg | 0.10 microgram |
| HG75 | 12.0 mg | 0.15 microgram |
| HG90 | 28.8 mg | 0.30 microgram |

All of the HG50, HG75 and HG90 prepared hereinabove exhibited significant effects in all of the following pharmacological tests and, accordingly, the result obtained for HG75 will be given as a representative thereof as hereinafter.

### [Actions]

### I. Immunomodulating Action:

The immunomodulating action was measured using the production of antibodies specific to trinitrophenyl (TNP) and also a delayed TNP-specific hypersensitivity (DTH) reaction as targets.

### (1) Preparation of Trinitrophenyl-Bonded Sheep Red Cells (TNP-SRBC).

Trinitrobenzenesulfonic acid (TNBS) was dissovled in a physiological saline solution buffered with phosphoric acid to prepare a solution (40 mg/7.0 ml; pH 7.2) and then 1 ml of sheep red cell pellets was added with stirring. The mixture was allowed to stand at room temperature with stirring several times with light-shielding, and washed with a physiological saline solution three times. Then it was centrifuged at 3,000 rpm for five minutes and made into a solution of 5 x 10⁹/ml using a physiological saline solution.

### (2) Production of a TNP-Specific Antibody.

TNP-SRBC (10⁹ cells) was intraperitoneally administered to male BALB/c mice of six to eight weeks age and the anti-TNP antibody in serum was measured by an enzymatic immunoassay (ELISA) using a dinitrophenyl-bovine serum albumin (DNP-BSA). The result was that a potent antibody production of anti-TNP-IgM and anti-TNP-IgG was noted having a peak on 4th to 6th days. Incidentally, in the case of BALB/c nude mice having no thymus, production of antibodies of both types was rarely noted.

### (3) TNP-Specific DTH Reaction.

Like in the above-mentioned (2), the mice were sensitized with TNP-SRBC and, on the 14th day, 0.025 ml of TNB (4.7 mg/ml) was injected into a right hind paw to induce a TNP-specific DTH reaction. After 24 hours and 48 hours from the induction, thickness of both paws was measured using a dial gauge and the difference in the thickness between the right and left paws was expressed as the intensity of the DTH reaction. The result was that, after 24 hours from the induction, a DTH reaction was clearly noted but, in the case of BALB/c nude mice, no DTH reaction was observed at all. Incidentally, this measuring system of the DTH reaction can be subjected to further tests using the same mice as in the case of the antibody production system of the above (2).

### (4) Measurement of the Action of the Tested Drugs.

The above-mentioned test system was used for checking the action of the histamine-added mouse gamma-globulin (150 mg/kg/day) (the effective ingredient of the present invention), ciclosporin A (100 mg/kg/day), cyclophosphamide (100 mg/kg/day), prednisolone (0.5 kg/kg/day) and levamizole (5 mg/kg/day) to the anti-TNP antibody production and to the TNP-specific DTH reaction by a hypodermic injection for four days from the sensitization with TNP-SRBC.

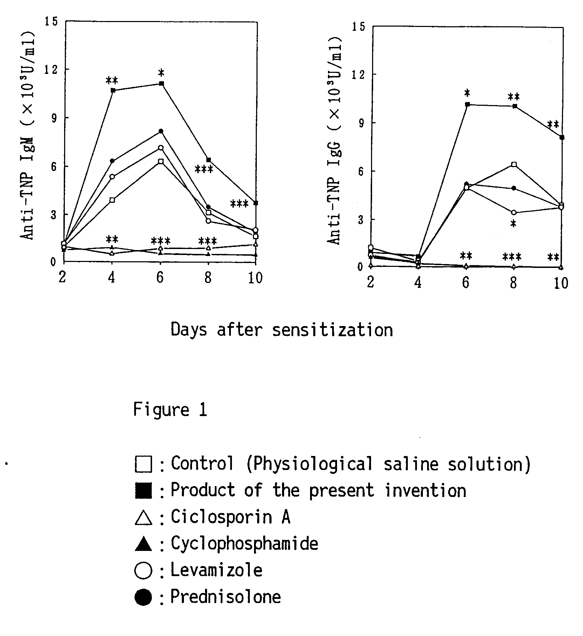

The result for the anti-TNP antibody production system is given in Fig. 1 while that for the TNP-specific DTH reaction system is given in Table 1.

Incidentally, in the following test results, significant difference in the average values from the control was calculated by means of Student's t-test and is expressed with asterisks (*: p < 0.05; **: p < 0.01: ***: p < 0.001).

**Table 1**

| Tested Compound | Swelling in Paws (x 10⁻²mm) | |
|---|---|---|
| | After 24 Hrs | After 48 Hrs |
| Not Sensitized | 11.7 ± 3.8*** | 10.0 ± 2.8* |
| Control | 49.2 ± 3.5 | 21.7 ± 3.8 |
| Product of this Invention | 26.7 ± 4.4** | 10.8 ± 2.4* |
| Ciclosporin | 15.8 ± 2.0*** | 10.8 ± 2.7* |
| Cyclophosphamide | 13.3 ± 2.1*** | 15.8 ± 2.4 |
| Prednisolone | 30.0 ± 5.6* | 8.3 ± 3.3* |
| Levamizole | 40.0 ± 6.5 | 20.0 ± 5.0 |

### II. Inhibitory Action to Hypereosinophilicity.

### (1) Hypereosinophilic Model Induced by Ragweed Pollen Antigen.

A ragweed pollen extract (which was diluted to an extent of 1,000 times using a physiological saline solution) was hypodermically injected to female BALB/c mice of six to eight weeks age for sensitization at the dose of 0.1 ml on the initiation day and on the first day and 0.2 ml on the sixth, eighth and fourteenth days. On the twentieth day, 0.2 ml of a 1,000 times diluted ragweed antigen was intraperitoneally injected to the mice to induce the reaction. 24th hours after the induction, the peritoneal exudate cells were recovered and subjected to a Giemsa staining and the total cell numbers, number of eosinophils, number of neutrophils and number of mononuclear cells were counted. As a result, the number of the eosinophils was at a peak after 24 hours from the induction. Incidentally, in the case of BALB/c nude mice having no T cells, no exduation to peritoneum was noted at all both in eosinophils and in neutrophils.

### (2) Measurement of the Action of the Tested Pharmaceuticals.

The above-mentioned hypereosilophilic models were used for checking the action to the hypereosinophilicity by a hypodermic injection of the effective ingredient of the present invention, i.e. a histamine-added mouse gamma-globulin, at a dose of 3 mg/mouse/day twice a week for three weeks until the induced day. A further test was conducted by administering the corresponding amount of each of histamine (dihydrochloride) and mouse gamma-globulin which are the constituting components of the histamine-added mouse gamma-globulin. Moreover, ciclosporin A which is known as an immunosuppressive agent was hypodermically administered at the dose of 100 mg/kg/day for three days from two days prior to the induction until the induced day and the results were used for comparison.

An example of the results is given in Table. 2.

**Table 2**

| Tested Pharmaceuticals | Nos of Eosinophils Exudated to Peritoneum (x 10⁵ cells) |
|---|---|
| Not Sensitized | 0.4 ± 0.1*** |
| Control | 3.7 ± 0.4 |
| Product of the Invention | 0.5 ± 0.1*** |
| Histamine | 3.4 ± 0.6 |
| gamma-Globulin | 3.2 ± 0.5 |
| Ciclosporin A | 0.4 ± 0.2*** |

### (3) Measurement of the Action of the Tested Pharmaceuticals Using Hypereosinophilic Model Induced by Platelet Activating Factor (PAF).

A PAF (5 micrograms/200 microliters) was intraperitoneally injected to female BALB/c mice of six to eight weeks age to induce the reaction. After 24th hours from the induction, the peritoneal exudate cells were recovered end the numbers of the eosinophils were counted. The resulting hypereosinophilic model was used for checking the suppressive action of the product of the present invention (i.e. histamine-added mouse gamma-globulin) against the eosinophilicity by the same manner as mentioned hereinabove.

An example of the results is given in Table 3.

**Table 3**

| Tested Pharmaceutical | Numbers of Exudated Eosinophils to Peritoneum (x 10⁵ cells) |
|---|---|
| Not Sensitized | 0.3 ± 0.1** |
| Control | 2.3 ± 0.5 |
| Product of the Invention | 0.8 ± 0.1* |
| Histamine | 2.2 ± 0.8 |
| gamma-Globulin | 1.5 ± 0.2 |

It is clear from the result of Fig. 1 that the histamine-added gamma-globulin (the product of the present invention) exhibited significant promoting action to the IgG and IgM antibody production. However, ciclosporin A and cyclophosphamide (immunosuppressive agents) significantly suppressed the production of both antibodies while prednisolone (an adrenocortical hormone) and levemizole (said to exhibit an immunomodulating action) gave no significant affection on the antibody production at the dose of the present test.

On the other hand, the product of the present invention exhibited a significant suppressive action to the delayed type hypersensitivity (DTH) as shown in Table 1 and, similarly, both cyclosporin A and cyclophosphamide showed clear suppressive actions. In addition, prednisolone showed a weak suppressive action while levemisole gave no significant affection.

As such, ciclosporin A and cyclophosphamide (conventional immunosupressive agents) significantly suppressed the immunoreactions of both IgG and IgM antibody production and the DTH reaction while histamine-added gamma-globulin (the effective ingredient of the present invention) exhibited a promoting action to the antibody production and a suppressive action to the DTH reaction. As such, it is now clear that the histamine-added gamma-globulin has an immunomodulating action which is clearly different from the conventional immunosuppressive agents.

Furthermore, as shown in Table 2, the product of the present invention significantly suppressed the eosinophil exudation into peritoneum in the hypereosinophilic model induced by a ragweed pollen antigen. Moreover, as apparent from the result of Table 3, the histamine-added gamma-globulin exhibited a clear suppressive action to the hypereosinophilicity induced by administration of PAF instead of by an antigen induction like ciclosporin A which is an immunosuppressive agent. It is clear that said suppressive action to the hypereosinophilicity is an action which is specific to the effective ingredient of the present invention since said action is not observed in each of the histamine and the gamma-globulin which are the components of the histamine-added gamma-globulin. Moreover, the product showed a significant suppressive action to the above-mentioned antigen-induced and PFA-induced hypereosinophilic models even with the same administration regime as in ciclosporin A (i.e. from two days before the induction until the induced day).

It will be clear from the above-mentioned result of the pharmacological tests that the pharmaceutical composition of the present invention has a specific immunomodulating action which is clearly different from conventional immunosuppressive agents. Accordingly, said pharmaceutical composition is useful as a pharmaceutical agent for the therapy of the diseases with abnormal immune system such as, for example, chronic articular rheumatism, systemic lupus erythematosus, multiple sclerosis, etc. and various types of immunodeficiency syndromes. In addition, since the product of the present invention exhibits a suppressive action to a hypereosinophilicity, it can be used as a therapeutic agent for infectious diseases, parasitic diseases, respirtory diseases, autoimmune diseases and eosinophilia caused by malignant tumor, etc. as well.

Eosinophils are known as effector cells which accumulate at the stimulated portion which causes inflammation end result in an inflammatory symptoms. Accordingly, the agents which suppress the increase in eosinophils can be used as the remedies for suppressing the inflammation. The pharmaceutical composition of the present invention containing a histamine-added gamma-globulin exhibits an action of suppressing the tumor in the DTH reaction in addition to the above-mentioned suppressive action to an increase in eosinophils and, accordingly, the composition is useful as an excellent antiinflammatory agent as well.

### (Examples)

As hereunder, an example of the formulation of the pharmaceutical composition according to the present invention will be given . It is preferred that the dose is suitably selected depending upon the type of the disease, degree of the disease, age and sex of the patient, term of the administration, etc.

| Formulation Example 1 | Components | Amount in a Vial |
|---|---|---|
| Injection (2 ml) | Histamine-Added Human gamma-Globulin | 36 mg |
| | Histamine Dihydrochloride | 0.45 microgram |
| | Sodium Chloride | q.s. |

### Brief Explanation of the Drawing:

Fig. 1 is a graph showing the potentiating action of the product of the present invention to the anti-TNP antibody production.

## Claims

1. The use of histamine-added gamma-globulin for the manufacture of a medicament effective against an autoimmune disease.

2. The use according to claim 1, wherein the disease is chronic articular rheumatism, systematic lupus erythematosis or multiple sclerosis.

3. The use of histamine-added gamma-globulin for the manufacture of a medicament effective against an immunodeficiency syndrome.

4. The use of histamine-added gamma-globulin for the manufacture of a medicament effective as a hypereosinophilicity suppressive agent.

5. The use according to claim 4, wherein the hypereosinophilicity suppressive agent is effective against infectious diseases, parasilic diseases, respiratory diseases, autoimmune diseases and cosinophilia caused by malignant tumor.

6. The use of histamine-added gamma-globulin for the manufacture of a medicament effective as an antiinflammatory agent.

## Patentansprüche

1. Verwendung von mit Histamin versetztem Gammaglobulin für die Herstellung eines Arzneimittels, das gegen Autoimmunerkrankungen wirksam ist.

2. Verwendung gemäß Anspruch 1, worin die Krankheit chronischer Gelenkrheumatismus, systemischer Lupus erythematodes oder Multiple Sklerose ist.

3. Verwendung von mit Histamin versetztem Gammaglobulin für die Herstellung eines Arzneimittels, das gegen Immundefizienz-Syndrom wirksam ist.

4. Verwendung von mit Histamin versetztem Gammaglobulin für die Herstellung eines Arzneimittels, das als die Hypereosinophilie unterdrückendes Mittel wirksam ist.

5. Verwendung gemäß Anspruch 4, worin das die Hypereosinophilie unterdrückende Mittel gegen Infektionskrankheiten, parasitäre Erkrankungen, Atemwegserkrankungen, Autoimmunerkrankungen und Eosinophilie, verursacht durch einen malignen Tumor, wirksam ist.

6. Verwendung von mit Histamin versetztem Gammaglobulin für die Herstellung eines Arzneimittels, das als antiinflammatorisches Mittel wirksam ist.

## Revendications

1. Utilisation de gamma-globuline additionnée d'histamine en vue de la fabrication d'un médicament efficace contre une maladie auto-immune.

2. Utilisation selon la revendication 1, dans laquelle la maladie est un rhumatisme articulaire chronique, un lupus érythémateux aigu disséminé ou une sclérose en plaques.

3. Utilisation de gamma-globuline additionnée d'histamine en vue de la fabrication d'un médicament efficace contre un syndrome d'immunodéficience.

4. Utilisation de gamma-globuline additionnée d'histamine en vue de la fabrication d'un médicament efficace en tant qu'agent dépresseur d'hyperéosinophilie.

5. Utilisation selon la revendication 4, dans laquelle l'agent dépresseur d'hyperéosinophilie est efficace contre des maladies infectieuses, des maladies parasitaires, des maladies respiratoires, des maladies auto-immunes et une éosinophilie provoquée par une tumeur maligne.

6. Utilisation de gamma-globuline additionnée d'histamine en vue de la fabrication d'un médicament efficace en tant qu'agent anti-inflammatoire.
